# EUROPEAN PATENT APPLICATION

(11) **EP 1 795 439 A2**
(43) Date of publication of application: **13.06.2007**
(21) Application number: 06125853.9
(22) Date of filing: 11.12.2006
(51) Int. Cl.: B62J 7/08

(54) **A bungee cord hook**

(30) Priority: 12.12.2005 US 749560 P
(71) Applicant: Master Lock Company LLC, Oak Creek WI 53154 (US)
(72) Inventor: van Handel, MARK, Madision, WI 53704 (US)
(74) Representative: Quintelier, Claude

(57) **Abstract**

A bungee cord hook. An embodiment of the bungee cord hook includes a base, a hook, and a safety clip. The hook has a generally straight portion connected to and extending away from the base and an arcuate portion connected to the straight portion and curving away from the base. The safety clip generally closes the bungee cord hook between the end of the hook and the base.

## Description

### Cross-Reference to Related Applications

This non-provisional application claims the benefit of U.S. Provisional Patent Application No. 60/749,560, entitled "Hook," filed December 12, 2005, which is hereby incorporated by reference in its entirety, to the extent it is not conflicting with the present application.

### Field of the Invention

The present invention relates to a hook for use with a bungee cord.

### Background of the Invention

Ropes, cords, belts, and various other tie-down devices are commonly used to secure cargo and other objects. One such known device is a bungee cord. Typically, a bungee cord includes a stretchable cord and a hook secured at either end. Bungee cords are adaptable to secure a variety of items in various spaces. In application, one of the hooks is fastened to a target or anchor point, and the stretchable cord is then secured over or around the object to be secured. The other hook is connected to a second target point, to maintain the cord in a taut position across or around the object to be secured.

Exemplary bungee cord hooks include a base portion and a curved hook portion. In application, a target object is secured between the base portion and the curved hook portion. Many cord designs center the cord attachment in-line with the hook. This orientation keeps the hook straight under tension but makes it difficult to secure the hook to the target because the user's hands can obstruct the hook. Further, conventional hook designs cause limited application for cords because of limited target selection.

### Summary of the Invention

A bungee cord hook having a base, a hook, and a safety clip. An embodiment of the invention includes a hook with a reversed orientation having a generally straight portion connected to and extending away from the base and an arcuate portion connected to the straight portion and curving away from the base. In addition, the safety clip generally closes the opening between the base and the hook to prevent the hook from slipping off an anchor when no tension is applied to the bungee cord. The present invention provides an improved bungee cord offering greater ease of use, added security, and wider application. Further features and advantages of the invention will become apparent from the following detailed description made with reference to the accompanying drawings.

### Brief Description of the Drawings

Figure 1 is a side view of a hook made in accordance with an embodiment of the present invention, showing a twin wire reverse hook portion and a safety clip;
Figure 2 is a front view of the hook of Figure 1;
Figure 3 is a side view of the hook of Figure 1, shown from the opposing side of Figure 1;
Figure 4 is a rear view of the hook of Figure 1;
Figure 5 is a top view of the hook of Figure 1;
Figure 6 is a bottom view of the hook of Figure 1;
Figure 7 is a perspective view of the hook of Figure 1;
Figure 8 is a perspective view of a hook made in accordance with another embodiment of the present invention, showing a spaced twin wire reverse hook portion and a safety clip;
Figure 9 is a side view of the hook of Figure 8;
Figure 10 is a front view of the hook of Figure 8;
Figure 11 is a side view of the hook of Figure 8, shown from the opposing side of Figure 9;
Figure 12 is a rear view of the hook of Figure 8;
Figure 13 is a top view of the hook of Figure 8;
Figure 14 is a bottom view of the hook of Figure 8;
Figure 15 is a perspective view of a bungee cord made in accordance with an embodiment of the present invention, showing two hooks of Figure 1 attached at either end of a stretchable cord;
Figure 16 is a perspective view of a hook made in accordance with another embodiment of the present invention, showing a twin wire reverse hook portion; and
Figure 17 is a perspective view of a hook made in accordance with another embodiment of the present invention, showing a spaced twin wire reverse hook portion.

### Detailed Description of the Invention

This Detailed Description of the Invention merely describes embodiments of the invention and is not intended to limit the scope of the claims in any way. Indeed, the invention as described is broader than and unlimited by the preferred embodiments, and the terms used have their full ordinary meaning.

The present invention of a hook will be described for use with a bungee cord. However, it should be understood that this is for exemplary purposes only and the invention can be applied to a wide variety of other products, such as for example, cables, ropes, and chains.

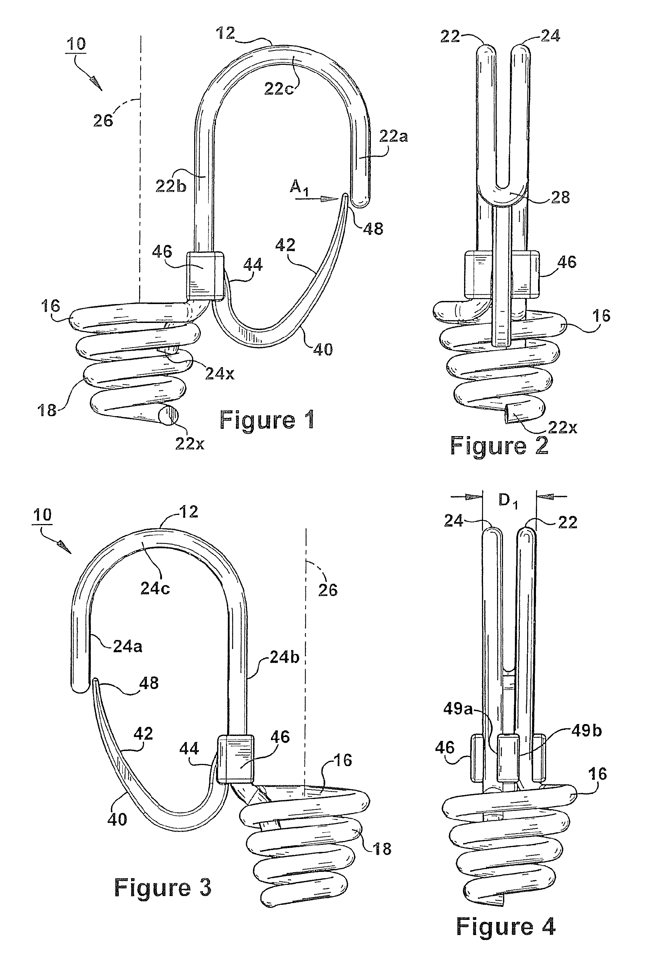

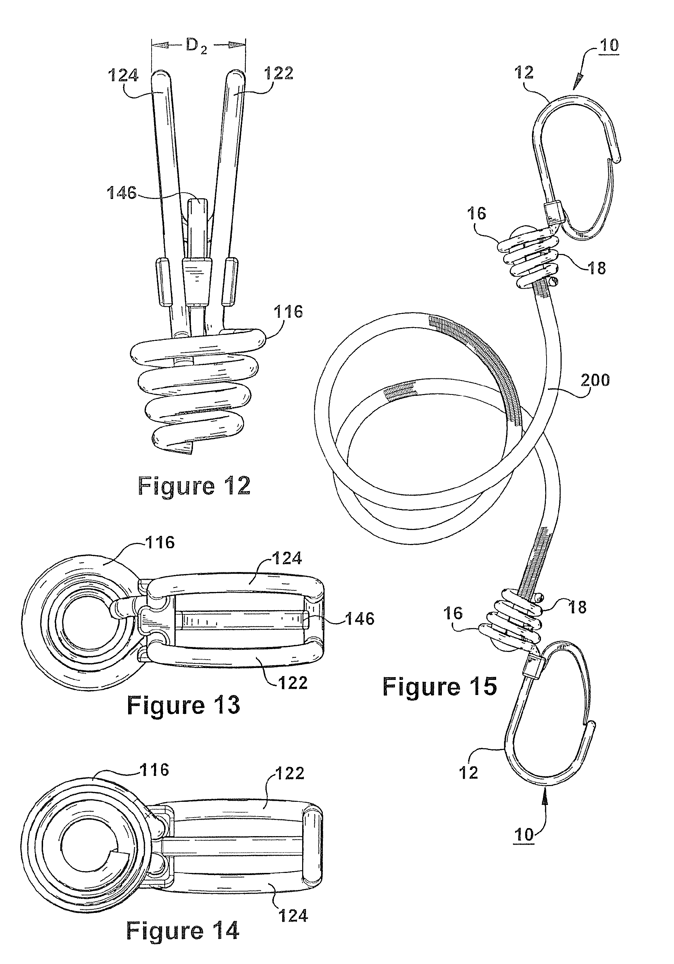

Referring now to the drawings, a reverse orientation hook is illustrated. The reverse orientation of the hook portion of the hook makes it easier for a user to place and attach the hook compared to conventional hook shapes. This ease of use is due at least, in part, to the opening of the hook portion 12 being unobstructed by the base portion 16. In "non-reversed" conventional bungee hooks, the body portion obstructs the opening of the hook. This is because conventional hooks curve in a direction towards the base as opposed to away from the base. Figure 1 is a side view of a hook 10 made in accordance with an embodiment of the present invention. As shown is Figure 1, the hook 10 includes a hook portion 12, a base portion 16, and a clip 40. The hook can be attached to a bungee cord 200 (shown in Figure 15) for use in securing cargo and other objects. The hook can be attached to the cord by any suitable method known in the art, such as for example, knotting or doubling over the end of the cord to prevent the hook from separating from the cord.

The hook portion 12 and base portion 16, as shown in Figures 1-7, are formed from a continuous piece of single wire. However, it should be apparent that the hook portion 12 and base portion 16 may be formed from two or more wires. The wire may be made of any material of suitable strength, such as for example, a rigid metal inner portion and a protective plastic coating applied on top of the metal portion, e.g., a plastic coated steel. The protective plastic coating applied to the present invention is a thick hardened, over-molded plastic that is less susceptible to tearing, cracking or exposure of the metal portion contained therein. Typically bungee cord hooks are metal and may, or may not, include a thin elastomeric coating. Such coating is susceptible to tearing or cracking and does not provide protection for the metal portion against possible environmental elements.

Referring to Figures 1-7, the hook portion 12 is formed in a reverse orientation with twin portions, i.e., a first twin portion 22 and a second twin portion 24. The hook portion 12 may also be formed from any number of portions, such as one, three, or four individual portions. The first and second twin portions 22, 24 each include short legs, 22a, 24a, respectively, and long legs, 22b, 24b, respectively. However, in an alternative embodiment, the first and second twin portions 22, 24 only include long legs 22b, 24b. The long legs 22b, 24b extend away from the base portion 16 and are generally parallel to an elongated axis 26 formed by a conical shaped series of coils 18. The first and second twin portions 22, 24 also include arcuate portions 22c, 24c, respectively, that connect the long legs 22b, 24b to the short legs 22a, 24a and curve away from the elongated axis 26 and the base portion 16. In an alternative embodiment, the arcuate portions 22c, 24c only connect to the long legs 22b, 24b. The twin portions 22, 24 are also connected together, forming the end 28 of the hook portion 12. The twin portions 22, 24 are generally parallel from each other at a spaced distance D₁, as shown in Figure 4. Alternatively, as shown in Figure 12, the twin portions 122, 124 are aparallel and the distance between the twin portions 122, 124 varies along the length of the twin portions 122, 124.

As mentioned, the hook portion 12 is formed from a single piece of continuous wire. As shown in Figures I and 2, the first portion 22 of the wire terminates at the distal end 22x of the base portion 16. The second portion 24 of the wire terminates at a point 24x within the coils of the base portion 16. It should be apparent that other termination points can be used in the practice of this invention.

The base portion 16 of the hook 10 is formed as a series of coils 18. The series of coils 18 forms a cavity 19 through which a bungee cord 200 (shown in Figure 15) is inserted and secured. As shown in Figures 1-7, the base portion 14 is generally conical shaped and decreases in diameter in a direction away from the hook portion 12. As mentioned, the conical shape of the coils 18 forms an elongated axis 26. The shape and size of the base may vary in the practice of this invention.

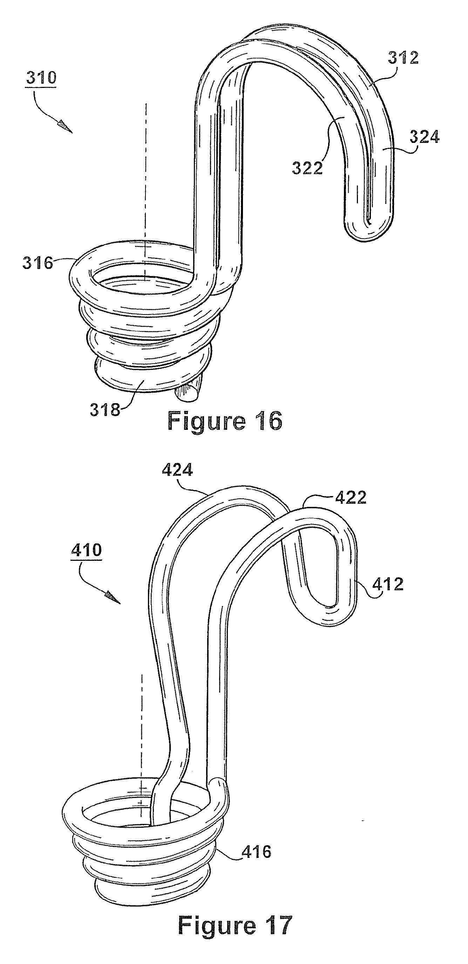

As shown in Figures 1-7, a clip 40 generally closes the opening between the end of the hook portion 12 and the base portion 16. It should be obvious to one with ordinary skill in the art, however, that the use of a clip is optional and not required to practice the invention. Exemplary hooks without clips are shown in Figures 16-17. The clip may be constructed of any material, such as a molded or stamped plastic, a stamped metal, or a wire form. The clip 40 includes an elongated portion 42, a proximal end 44, and a distal end 48. The proximal end 44 of the clip 40 is attached to the hook portion 12, but may alternatively be attached to the base portion 16. In addition, the distal end 48 may, or may not, interact with the hook portion 12 to create a positive lock. The elongated portion 42 can vary in shape, such as for example, straight or arcuate. The elongated portion 42, as shown, is arcuate, first curving down and away from the hook portion 12 then towards the end of the hook portion 12. In an alternative embodiment, the elongated portion 42 is arcuate and reversed, first curving up and towards the hook portion 12 and then towards the end of the hook portion 12.

As shown in Figures 1-7, the proximal end 44 of the clip is attached to a base 46 formed to snap onto the hook portion 12. As shown, the base 46 is removable from the hook portion 12. However, the base 46 may be permanently attached. In an alternative embodiment, the base 46 attaches to the coils 18 of the base portion 16. Figure 4 is a rear view of the hook 10 of Figure 1, showing two slots 49a, 49b that are shaped in the rear of the base 46 to be press fit onto the second twin portion 24 and first twin portion 22, respectively. Alternatively, any number of slots may be used to attach the base 46 to either the hook portion 12 or the base portion 16 of the hook 10. It should also be apparent that other methods and structures for clip 40 attachment may be used in the practice of this invention.

As shown in Figure 1, the clip 40 may also be biased in a direction A₁ towards the end 28 of the hook portion 12. The biased clip 40 may, or may not, apply a force against the hook portion 12. The biased clip 40 prevents the hook 10 from slipping off an anchor when no tension is on the cord. The configuration of the clip 40 is one way to create the biasing effect. Exemplary clip 40 configurations include combinations of material, such as for example, a pliable plastic, and shape. However, it should be apparent that any biasing method or structure may be used to practice this invention, such as for example, a spring.

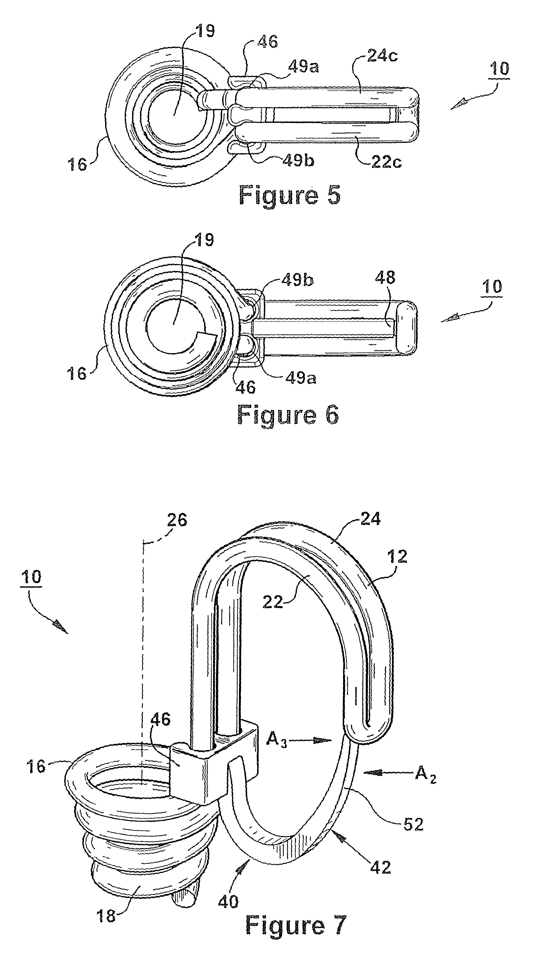

A perspective view of the hook of Figure 1 is illustrated in Figure 7. The hook 10 may be attached to either end of a bungee cord 200 (shown in Figure 15). During application, a user stretches the cord until the hook 10 is proximate to an anchor (not shown), such as for example, a hasp or loop. By pressing the outer surface 52 of the elongated member 42 against the anchor, the clip 40 moves in a direction A₂ relative to the base portion 16, effectively enclosing the anchor within the hook portion 12. After the anchor is within the hook portion 12, biasing forces move the clip 40 in a direction A₃ relative to the base portion 16 to effectively secure the hook 10 to the anchor.

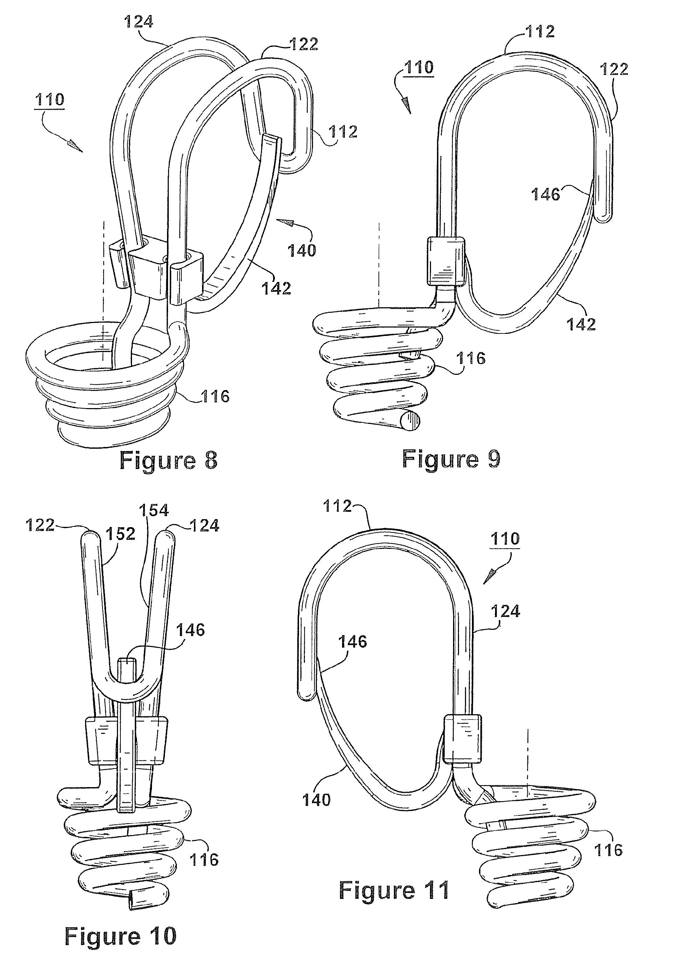

Figures 8-14 illustrate a hook 1 10 made in accordance with another embodiment of the present invention. The hook 110 has a reverse orientation with a spaced twin wire portion. As shown in Figures 8-11, the hook 110 includes a hook portion 112, a base portion 116, and a clip 140. The base portion 116 is similar to the base portion 16 of the hook 10 shown in Figures 1-7.

Referring to Figures 8-14, the hook portion 112 is formed in a reverse orientation with twin portions, i.e., first twin portion 122 and second twin portion 124. As best shown in Figure 12, the first and second twin portions 122, 124 are aparallel and spaced apart from each other at a distance D₂. This spaced positioning creates a channel defined by the inner surfaces 152, 154, respectively, of the first twin portion 122 and second twin portion 124. The channel may accommodate certain objects being secured to the hook 10, such as for example, knobs or protruding corners. This channel also allows for an increased length of an elongated member 142 to secure the extending distal end 146 of the clip 140.

Figure 16 is a perspective view of a hook 310 made in accordance with another embodiment of the present invention. As shown in Figure 16, the hook 310 comprises a twin wire reverse hook portion 312. The twin portions 322, 324 of the hook portion 312 are generally parallel to each other. However, the hook 310 shown in Figure 16 does not include a clip. As stated earlier, the clip is removable and is not required to practice the invention. Similarly, Figure 17 is a perspective view of a hook 410 showing a spaced twin wire reverse hook portion 412. As shown in Figure 17, the twin portions 422, 424 are aparallel and the distance between the twin portions 422, 424 varies along the length of the twin portions 422, 424. The hook 410 shown in Figure 17 also does not include a clip.

While several embodiments of the invention has been illustrated and described in considerable detail, the present invention is not to be considered limited to the precise constructions disclosed. Various adaptations, modifications and uses of the invention may occur to those skilled in the arts to which the invention relates. It is the intention to cover all such adaptations, modifications and uses falling within the scope or spirit of the claims filed herewith.

## Claims

1. A bungee cord hook, comprising:
a base portion;
a hook portion having a generally straight portion connected to and extending away from the base portion and an arcuate portion connected to the straight portion and curving away from the base portion; and
a clip generally closing the bungee cord hook between an end of the hook portion and the base portion.

2. The bungee cord hook of claim 1, wherein the base portion further comprises a series of coils.

3. The bungee cord hook of claim 2, wherein the series of coils form a conical shape having an elongated axis, wherein the straight portion of the hook portion is parallel to the elongated axis.

4. The bungee cord hook of claim 2, wherein the series of coils form a cavity for securing a bungee cord.

5. The bungee cord hook of claim 1, wherein the base portion and the hook portion further comprise a continuous piece of material.

6. The bungee cord hook of claim 1, wherein the base portion and the hook portion further comprise a continuous piece of material having a rigid metal inner portion and a protective plastic coating applied on top of the metal inner portion.

7. The bungee cord hook of claim 1, wherein the hook portion further comprises two twin portions, each twin portion having a straight portion extending away from the base portion and an arcuate portion connected to the straight portion curving away from the base portion.

8. The bungee cord hook of claim 7, wherein the arcuate portions of the twin portions are generally parallel to each other.

9. The bungee cord hook of claim 7, wherein the arcuate portions of the twin portions are aparallel to each other.

10. The bungee cord hook of claim 7, wherein each twin portion and the base portion comprise a continuous piece of material.

11. The bungee cord hook of claim 1, wherein the clip comprises a proximal end, an arcuate portion, and a distal end.

12. The bungee cord hook of claim 1, wherein the clip contacts the end of the hook portion creating a positive lock.

13. The bungee cord hook of claim 1, wherein a proximal end of the clip attaches to a clip base.

14. The bungee cord hook of claim 13, wherein the clip base is removably mounted to the hook portion.

15. The bungee cord hook of claim 13, wherein the clip base further comprises a slot for attaching the clip to the hook portion.

16. The bungee cord hook of claim 1, wherein the clip extends from the straight portion of the hook portion.

17. The bungee cord hook of claim 1, wherein a configuration of the clip biases a distal end of the clip towards the end of the hook portion.

18. A bungee cord hook, comprising:
a base portion;
a hook portion having two twin portions, each twin portion comprising a proximal end and a distal end, wherein the proximal end of at least one twin portion connects to the base portion and the distal end of each twin portion connects to each other; and
a clip generally closing the bungee cord hook between an end of the hook portion and the base portion.

19. The bungee cord hook of claim 18, wherein the base portion further comprises a series of coils forming a cavity for securing a bungee cord.

20. The bungee cord hook of claim 19, wherein the series of coils form a conical shape determining an elongated axis.

21. The bungee cord hook of claim 20, wherein each twin portion comprises a straight portion extending away from the base portion and parallel to the elongated axis of the conical shaped base.

22. The bungee cord hook of claim 18, wherein the base portion and the hook portion further comprise a continuous piece of material comprising a rigid metal inner portion and a protective plastic coating applied on top of the metal inner portion.

23. The bungee cord hook of claim 18, wherein each twin portion comprises a straight portion extending away from the base portion and an arcuate portion connected to the straight portion curving away from the base portion.

24. The bungee cord hook of claim 23, wherein the arcuate portions of the twin portions are generally parallel to each other.

25. The bungee cord hook of claim 23, wherein the arcuate portions of the twin portions are aparallel to each other.

26. The bungee cord hook of claim 18, wherein the clip comprises a proximal end, an arcuate portion, and a distal end.

27. The bungee cord hook of claim 18, wherein the clip contacts the end of the hook portion creating a positive lock.

28. The bungee cord hook of claim 18, wherein a proximal end of the clip attaches to a clip base.

29. The bungee cord hook of claim 28, wherein the clip base is removably mounted to the hook portion.

30. The bungee cord hook of claim 28, wherein the clip base further comprises a slot for attaching the clip to the hook portion.

31. The bungee cord hook of claim 18, wherein a configuration of the clip biases a distal end of the clip towards the end of the hook portion.

32. A bungee cord hook, comprising:
(a) a base portion having a series of coils formed from a single piece of material, wherein the series of coils form a cavity for securing a bungee cord;
(b) a hook portion having two twin portions formed from the piece of material, wherein each twin portion comprises:
(i) a proximal end and a distal end, wherein the distal end of each twin portion connects to each other;
(ii) a first straight portion extending away from the base portion;
(iii) an arcuate portion curving away from the base portion, wherein the twin portions are spaced a distance apart along the length of the arcuate portions; and
(iv) a second straight portion generally parallel to the first straight portion; and
(c) a clip having a proximal end, a distal end, an arcuate portion, and a clip base disposed at the proximal end and generally closing the bungee cord hook between an end of the hook portion and the base portion, wherein the clip base comprises two slots for removably mounting the clip to the hook portion.

33. The bungee cord hook of claim 32, wherein the proximal end of at least one twin portion connects to the base portion.

34. The bungee cord hook of claim 32, wherein the series of coils form a conical shape having an elongated axis.

35. The bungee cord hook of claim 32, wherein the clip creates a springing effect biasing the distal end of the clip towards the distal end of each twin portion.

36. A bungee cord, comprising:
(a) a stretchable cord; and
(b) a bungee cord hook connected at an end of the stretchable cord, wherein the bungee cord hook comprises
(i) a base portion,
(ii) a hook portion having two twin portions, each twin portion comprising a proximal end and a distal end, wherein the distal end of each twin portion connects to each other, and
(iii) a clip generally closing the bungee cord hook between an end of the hook portion and the base portion.

37. A bungee cord hook, comprising:
(a) a base portion having a series of coils formed from a single piece of material, wherein the series of coils form a cavity for securing a bungee cord; and
(b) a hook portion having two twin portions formed from the piece of material, wherein each twin portion comprises:
(i) a proximal end and a distal end, wherein the distal end of each twin portion connects to each other;
(ii) a straight portion extending away from the base portion; and
(iii) an arcuate portion curving away from the base portion, wherein the twin portions are spaced a distance apart along the length of the arcuate portions.

38. The bungee cord hook of claim 37, wherein the bungee cord hook further comprises a clip removably mounted to the hook portion and generally closing the bungee cord hook between an end of the hook portion and the base portion.

39. The bungee cord hook of claim 37, wherein the series of coils form a conical shape having an elongated axis, wherein the straight portion of the hook portion is parallel to the elongated axis.

40. The bungee cord hook of claim 37, wherein the proximal end of at least one twin portion connects to the base portion.

41. The bungee cord hook of claim 37, wherein the arcuate portions of the twin portions are generally parallel to each other.

42. The bungee cord hook of claim 37, wherein the arcuate portions of the twin portions are aparallel to each other.
